# EUROPEAN PATENT SPECIFICATION

(11) **EP 0 692 469 B1**
(45) Date of publication and mention of the grant of the patent: **22.10.1997**
(21) Application number: 95115614.0
(22) Date of filing: 30.07.1993
(51) Int. Cl.: C07C 259/04, C07C 271/06

(54) **Process for recovering N,O-dialkylhydroxycarbamic acid ester**
Verfahren zur Isolierung von N,O-Dialkyl-hydroxycarbamin-Säureestern
Procédé pour l'isolement d'esters de l'acide N,O-dialkyl-hydroxycarbamique

(30) Priority: 26.03.1993 JP 68664/93
(43) Date of publication of application: 17.01.1996
(62) Divisional of application: 93306069.1
(73) Proprietor: MITSUI PETROCHEMICAL INDUSTRIES, LTD., Tokyo (JP)
(72) Inventor: Inoki, Satoshi, c/o Mitsui Petrochem. Ind., Ltd., Kuga-gun, Yamaguchi-ken (JP); Takesue, Mitsuyuki, Mitsui Petrochem. Ind., Ltd., Kuga-gun, Yamaguchi-ken (JP); Hashimoto, Isao, c/o Mitsui Petrochem. Ind., Ltd., Kuga-gun, Yamaguchi-ken (JP); Kihara, Noriaki, c/o Mitsui Petrochem. Ind., Ltd., Kuga-gun, Yamaguchi-ken (JP); Sugi, Kiyoaki, c/o Mitsui Petrochem. Ind., Ltd., Kuga-gun, Yamaguchi-ken (JP)
(74) Representative: Cresswell, Thomas Anthony

(56) References cited:
- DE-A- 3 245 503

## Description

This invention relates to a process for recovering an N,O-dialkylhydroxycarbamic ester. dimethylhydroxy.

A generally known process for preparing N,O-dialkylhydroxycarbamic acid ester from hydroxylamine or its salts comprises reacting chloroformic acid ester with a hydroxylamine salt in the presence of sodium hydroxide and subsequently reacting the reaction solution with a dialkyl sulfate in the presence of sodium hydroxide.

An example of a method for recovering the N,O-dimethylhydroxycarbamic acid ester comprises extracting the N,O-dimethylhydroxycarbamic acid ester with an organic solvent such as a halogenated hydrocarbon. From the viewpoint of environmental problems, however, a more effective method is desired. In addition, because O-methylhydroxylcarbamic acid ester, which is formed as an impurity in the course of the formation of the N,O-dimethylhydroxycarbamic acid ester, is also extracted, O-methylhydroxyamine is contained in the N,O-dimethylhydroxylamine obtained after deprotection. The boiling points of these compounds are close, ie 42.3°C for N,O-dimethylhydroxylamine and 48.1°C for O-methylhydroxylamine. Thus it is very difficult to purify them by distillation. A multi-stage distillation column is required for separation.

The present invention seeks to provide a process for recovering an N,O-dialkylhydroxycarbamic ester as an intermediate for N,O-dialkylhydroxylamine selectively, more readily and safely than the conventional processes.

The present invention provides a process for recovering an N,O-dialkylhydroxycarbamic acid ester of formula (III) 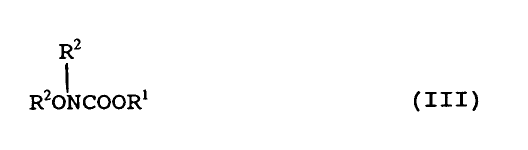 wherein R¹ is a hydrocarbon group, and R² is lower alkyl, said process comprising azeotropically distilling the N,O-dialkylhydroxycarbamic acid ester of formula (III) with water.

The N,O-dialkylhydroxycarbamic acid ester is, for example, methyl N,O-dimethylhydroxycarbamate.

The process of the present invention may be used to recover an N,O-dialkylhydroxycarbamic acid ester of formula (III) by azeotropically distilling the N,O-dialkylhydroxycarbamic acid ester of formula (III) with water from a solution containing the N,O-dialkylhydroxycarbamic acid ester of formula (III) and an O-alkylhydroxycarbamic acid ester of formula (IV)

R²ONHCOOR¹ (IV)

wherein R¹ and R² are as defined above.

The N,O-dialkylhydroxycarbamic acid ester is, for example, methyl N,O-dimethylhydroxycarbamate.

The N,O-dialkylhydroxycarbamic acid ester of formula (III) may be prepared by reacting hydroxylamine of formula NH₂OH or a salt thereof with a dihydrocarbyl carbonate of formula (I)

R¹OCOOR¹ (I)

wherein R¹ is as defined above,
in the presence of a base, and alkylating the resultant hydroxycarbamic ester of formula (II)

HONHCOOR¹ (II)

wherein R¹ is as defined above,
with an alkylating agent in the presence of a base.

The dihydrocarbyl carbonate may, for example, be dimethyl carbonate, the hydroxycarbamic acid ester may, for example, be methyl hydroxycarbamate and the N,O-dialkylhydroxycarbamic acid ester may, for example, be methyl N,O-dimethylhydroxycarbamate.

The preferred reaction temperatures in the carbamoylation and dialkylation are 5 ± 5°C and the pHs of the reaction solutions in the reactions are preferably 12 - 13.

The N,O-dialkylhydroxycarbamic acid ester of formula (III) may be hydrolysed in an aqueous solution or a solvent containing water in the presence of an alkali to prepare a N,O-dialkylhydroxylamine of formula (V)

R²ONHR² (V)

wherein R² is as defined above.

Examples of the hydrocarbon group represented by R¹ are C₁₋C₅ alkyl groups such as a methyl group, an ethyl group, a n-propyl group, an isopropyl group, a n-butyl group, an isobutyl group, a sec-butyl group, a tert-butyl group or a n-pentyl group, preferably C₁₋C₃ alkyl groups such as a methyl group, an ethyl group, a n-propyl group or an isopropyl group; C₃₋C₇ cycloalkyl groups such as a cyclopropyl group, a cyclobutyl group, a cyclopentyl group, a cyclohexyl group or a cycloheptyl group; aryl groups such as a phenyl group, a tolyl group or a xylyl group; and aralkyl groups such as a benzyl group or a phenethyl group.

The lower alkyl group represented by R² is a C₁₋C₄ alkyl group, for example a methyl group, an ethyl group, a n-propyl group, an isopropyl group, a n-butyl group, an isobutyl group, a sec-butyl group or a tert-butyl group.

Examples of the hydroxylamine or the salt thereof used as a starting material are hydroxylamine, hydroxylamine sulfate and hydroxylamine hydrochloride. Hydroxylamine sulfate and hydroxylamine hydrochloride are particularly preferable from the viewpoint of stability and price. The hydroxylamine or salts may be in the form of an aqueous solution or an organic solution such as in methanol.

Examples of the dihydrocarbyl carbonate of formula (I) are dialkyl carbonates such as dimethyl carbonate, diethyl carbonate, n-propyl carbonate or diisopropyl carbonate; dicycloalkyl carbonates such as dicyclopentyl carbonate, dicyclohexyl carbonate or dicycloheptyl carbonate; diaryl carbonates such as diphenyl carbonate, ditolyl carbonate or dixylyl carbonate; and diaralkyl carbonates such as dibenzyl carbonate or diphenetyl carbonate. In case of, for example, preparing methyl N,O-dimethylhydroxycarbamate, dimethyl carbonate is used.

Examples of a base are hydroxides of alkali metals or alkaline earth metals such as sodium hydroxide, potassium hydroxide, lithium hydroxide, cesium hydroxide and barium hydroxide; hydrogencarbonates of alkali metals such as sodium hydrogencarbonate and potassium hydrogen carbonate; carbonates of alkali metals or alkaline earth metals such as sodium carbonate, potassium carbonate and barium carbonate; and aluminate compounds such as sodium aluminate and potassium aluminate. These may be used independently or in combination. Among these bases sodium hydroxide is particularly preferable from the viewpoint of price. It is preferred that these bases are used in the form of an aqueous solution. However, they may be used as in the solid state or may be dissolved in an organic solvent such as an alcohol.

Examples of the alkylating agent are dialkylsulfuric acid esters such as dimethyl sulfate and diethyl sulfate; alkyl chlorides such as methyl chloride, ethyl chloride, propyl chloride and butyl chloride; alkyl bromide such as methyl bromide, ethyl bromide, propyl bromide and butyl bromide; and alkyl iodide such as methyl iodide, ethyl iodide, propyl iodide and butyl iodide. Dialkylsulfuric acid ester is preferable from the viewpoint of price. In case, for example, of preparing methyl N,O-dimethylhydroxycarbamate, it is preferred to use dimethyl sulfate.

As a reaction solvent , it is preferred to use water. However, inert organic solvents, for example, ethers such as diethyl ether and tetrahydrofuran; and aromatic compounds such as benzene and toluene may be used independently, in combination or as a two-layer system.

The molar ratio of the hydroxylamine or its salt to the dihydrocarbyl carbonate of formula (I) is generally 1:0.5 - 1:2, preferably 1:1 - 1:1.5, more preferably 1:1 - 1:1.3.

The molar ratio of the hydroxylamine or its salt to the alkylating agent is generally 1:2 or more. In case of using dimethyl sulfate as the alkylating agent, the molar ratio is preferably 1:2 - 1:4, more preferably 1:2 - 1:2.5.

There is no particular restriction on the quantity of the base to be used. However, it is preferably used in such a quantity as to control the pH of the reaction solution to within the range given later.

Although there is no particular restriction on the method for adding the starting materials, a method comprising firstly loading the hydroxylamine or its salt and a reaction solvent into a reactor and subsequently supplying the dihydrocarbyl carbonate of formula (I) and a base simultaneously is preferable because the reaction temperature and the pH of the reaction solution can thus be controlled to be constant. In case of using the sulfate or hydrochloride of hydroxylamine as a starting material, it is preferred to neutralize the salt by adding the base before supplying the dihydrocarbyl carbonate of formula (I).

In the dialkylation it is preferred to adopt a method for simultaneously supplying the alkylating agent and the base in order to control the reaction temperature and the pH of the reaction solution to be constant.

The hydroxycarbamic acid ester of formula (II) may be isolated as an intermediate on the way, or the two reactions, carbamoylation and dialkylation, may be continuously carried out using the same reactor without isolating the intermediate. In consideration of the reaction time and cost for extraction solvent, it is preferred that the two reactions are carried out using the same reactor without performing the isolation on the way.

The reaction temperature range at the time of carbamoylation or dialkylation is generally from -20 to 80°C, preferably from -10 to 40°C, more preferably from 0 to 25°C.

The pH range of the reaction solution at this time is generally 7 - 14, preferably 11 - 14, more preferably 12 - 14.

A combination of a reaction temperature of 5 ± 5°C with a reaction solution pH of 12 - 13 is optimum in case of industrially preparing the desired product not only because the yield is high but also because the reaction temperature and the reaction solution pH can be easily controlled.

The reaction pressure is generally atmospheric pressure or higher. In case that an alkylating agent vaporizes under the reaction conditions, it is required to carry out the reaction under pressure.

Examples of the N,O-dialkylhydroxycarbamic acid ester of formula (III) are N,O-dimethylhydroxycarbamic acid esters such as methyl N,O-dimethylhydroxycarbamate, ethyl N,O-dimethylhydroxycarbamate, propyl N,O-dimethylhydroxycarbamate or butyl N,O-dimethylhydroxycarbamate; N,O-diethyl hydroxycarbamic acid ester such as methyl N,O-diethylhydroxycarbamate, ethyl N,O-diethylhydroxycarbamate, propyl N,O-diethylhydroxycarbamate or butyl N,O-diethylhydroxycarbamate; N,O-dipropylhydroxycarbamic acid ester such as methyl N,O-dipropylhydroxycarbamate, ethyl N,O-dipropylhydroxycarbamate, propyl N,O-dipropylhydroxycarbamate or butyl N,O-dipropylhydroxycarbamate; and N,O-dibutylhydroxycarbamic acid ester such as methyl N,O-dibutylhydroxycarbamate, ethyl N,O-dibutylhydroxycarbamate, propyl N,O-dibutylhydroxycarbamate or butyl N,O-dibutylhydroxycarbamate. Methyl N,O-dimethylhydroxycarbamate is particularly desired.

In the above process for preparing N,O-dialkylhydroxycarbamic acid esters, the dihydrocarbyl carbonate of formula (I) is not only difficult to hydrolyze but is also unreactive with the hydroxy group of hydroxycarbamic acid esters as formed as intermediates. Therefore, the desired N,O-dialkylhydroxycarbamic acid ester can be obtained in high yields.

The present inventors have found that the ester of formula (III) has an azeotropic point with the water existing in the reaction system and that unreacted starting materials or O-alkylhydroxycarbamic acid ester of formula (IV) as impurities did not become contained in the azeotropic composition when the desired ester of formula (III) was directly distilled.

Hereinafter, the process for recovering the N,O-dialkylhydroxycarbamic acid ester of formula (III) will be detailed.

A reaction mixture to be subjected to the method of the present invention, as previously mentioned, for example in the process for preparing an N,O-dialkylhydroxycarbamic acid ester, is obtained by firstly reacting a hydroxylamine salt such as hydroxylamine sulfate with dihydrocarbyl carbonate in the presence of a base such as sodium hydroxide and subsequently reacting the reaction product with an alkylating agent such as dimethyl sulfate or methyl bromide. The reaction mixture contains an N,O-dialkylhydroxycarbamic acid ester. Previously the N,O-dialkylhydroxycarbamic acid ester has been isolated by adding an organic solvent such as chloroform, methylene chloride or ethylene dichloride to this reaction mixture to extract said ester and subsequently fractionally distilling the same.

The present invention provides a process for recovering the N,O-dialkylhydroxycarbamic acid ester comprising azeotropically distilling the N,O-dialkylhydroxycarbamic acid ester with water to recover said ester, without extracting the ester from the reaction mixture using an organic solvent as described above.

The present invention also include a process for recovering the N,O-dialkylhydroxycarbamic acid ester comprising azeotropically distilling the N,O-dialkylhydroxycarbamic acid ester with water from a solution containing inorganic salts such as sodium sulfate or sodium chloride, which are by-products formed in the preparation of the N,O-dialkylhydroxycarbamic acid ester, unreacted reagents and O-alkylhydroxycarbamic acid esters formed as impurities. This process selectively and easily purifys and distills said ester.

Hereinafter, a preferred process for recovery according to the present invention will be described more specifically.

N,O-dialkylhydroxycarbamic acid ester can be prepared according, for example, to the process described above.

The present invention is characterized by making the N,O-dialkylhydroxycarbamic acid ester form an azeotrope with water from a solution in its preparing step and subsequently distilling the N,O-dialkylhydroxycarbamic acid ester. The quantity of water necessary to form an azeotrope is generally the compositional quantity of an azeotrope of water and the N,O-dialkylhydroxycarbamic acid ester. That is, under atmospheric or reduced pressure distillation conditions, the quantity of water to ester by weight ratio is generally 1.0:1 or more, preferably 1.0-1.1:1.

The water used for forming the above azeotrope is generally supplied by adding a quantity necessary for azeotropic composition at the time of distillation. However, water which already exists in the mixture may be used. The mixture generally is a homogeneous system containing organic compounds such as an alcohol and inorganic compounds. However, it does not matter if the mixture is a heterogenous system containing other organic and inorganic compounds.

A preferred distillation apparatus has a distillation column having a fractional distilling function such as an Oldershow distillation column, that is an apparatus which enables selective recovery of azeotropic compositions alone. However, it is possible to use a batch system apparatus and a continuous system apparatus, each having a function of simultaneously recovering other compounds and azeotropic compositions.

The pressure in the above distillation may be atmospheric pressure, increased pressure or reduced pressure. Generally, atmospheric pressure or reduced pressure is adopted. The pressure range is preferably 1 - 760 mmHg, more preferably 10 - 250 mmHg. The distillation is generally carried out at a boiling point temperature where an azeotrope is formed at a set pressure. The distillation temperature, though it is preferably from 20 to 160°C, more preferably from 30 to 80°C, may exceed the boiling point temperature where an azeotrope is formed at a set pressure.

According to the method described above, it is possible to selectively recover a N,O-dialkylhydroxycarbamic acid ester more easily and safely than the conventional methods by azeotropically distilling said ester with water, without extracting the ester with an organic solvent.

An N,O-dialkylhydroxylamine of formula (V) can be prepared by hydrolyzing the N,O-dialkylhydroxycarbamic acid ester of formula (III) with acids such as hydrochloric acid or sulfuric acid. Free anhydrous N,O-dialkylhydroxylamine can be obtained without forming a N,O-dialkylhydroxylamine salt by carrying out hydrolysis using a catalytic quantity of an alkali instead of acids according to an industrial method.

When azeotropically distilling a crude product of an N,O-dialkylhydroxycarbamic acid ester of formula (III) with water according to the present recovery process, O-alkylhydroxycarbamic acid esters existing as impurities can be removed almost completely and thus the N,O-dialkylhydroxylamine or a salt thereof eventually produced is not contaminated with O-alkylhydroxylamine or a salt thereof after hydrolyzing the resulting product. Therefore, purification procedures are unnecessary.

### EXAMPLES

The present invention will now be further described in the following Examples.

### Example 1

41.03g (0.485 mol) of 95% hydroxylamine sulfate and 125m of water were added to a flask. The internal temperature of the flask was then cooled to 5°C. In an atmosphere of nitrogen, a 50% sodium hydroxide solution was dropped into the above solution from a dropping funnel such that the pH of the reaction solution reached 12. Then, maintaining the internal temperature at 5°C and the pH of the reaction solution within the range of 12 - 13, 49.5g (0.52 mol) of 98% dimethyl carbonate and a 50% sodium hydroxide solution were simultaneously supplied into the flask from the dropping funnels over a period of 40 minutes. After the completion of supply, the mixture was stirred for 30 minutes under the same conditions. Thereafter, maintaining the reaction temperature within the range of 5 - 8°C and the pH of the reaction solution within the range of 12 - 13, 139.0g (1.05 mol) of 95% dimethyl sulfate and a 50% sodium hydroxide solution were simultaneously supplied into the flask from dropping funnels over a period of 1.5 hours. After the completion of supply, the mixture was stirred for 3 hours under the same conditions. Then, the reaction temperature was raised to 50°C, and the reaction solution was stirred for 30 minutes while maintaining the pH thereof at 7.3 by dropping a saturated sodium hydrogencarbonate solution to decompose excessive dimethyl sulfate. After cooling to room temperature, the reaction solution was quantitatively analyzed by gas chromatography. It was found that 54.9g (yield, 95%) of methyl N,O-dimethylhydroxycarbamate was formed. Then, a five-stage Oldershow distillation column was attached to the flask to distill the reaction solution under a reduced pressure of 50 - 80 mmHg and at a boiling point of 40 - 50°C. 181.3g (purity, 29.0%; isolation yield, 92.5%) of an azeotropic distillate with water containing methanol and methyl N,O-dimethylhydroxycarbamate was obtained. N,O-dimethylhydroxylamine hydrochloride as the final product can be obtained by adding hydrochloric acid to this azeotropic distillate to hydrolyze the same and then evaporating the hydrolysate to dryness.

### Example 2

The reaction was carried out in the same manner as in Example 1, except that the distillation was carried out at atmospheric pressure and at a boiling point of 90 - 105°C instead of a reduced pressure of 50 - 80 mmHg and a boiling point of 40 - 50°C. 183.1g (purity, 24%; isolation yield, 77%) of an azeotropic distillate with water containing methanol and methyl N,O-dimethylhydroxycarbamate was obtained.

### Referential Examples 1 - 4

After cooling a reaction mixture containing 109.7g of methyl N,O-dimethylhydroxycarbamate obtained under the same conditions as in Example 1, except that the quantity of hydroxylamine sulfate used was 0.95 mol, to room temperature, 500ml of methylene chloride was added thereto. The mixture was stirred for 30 minutes and then transferred to a separation funnel. After leaving the funnel stationary for 30 minutes, the oily layer (the lower layer) was extracted. After repeating the extraction in the same manner using 500ml of methylene chloride, the extracted oily layers were combined and washed with 100ml of water. About a 500ml portion of the washed oily layer was loaded into a 1-l round-bottom flask equipped with a 60-mm Vigraux fractional column and then heated in a water bath (47 - 52°C) to distill methylene chloride away. Using a supply pipe, the remaining washed oily layer was continuously fed to the column to similarly distill methylene chloride away. The concentrate was transferred to a 300-ml round-bottom flask equipped with a five-stage Oldershow distillation column and superfractionated to give 108.7g (purity, 95.2%) of methyl N,O-dimethylhydroxycarbamate as a distillate. The total yield was 91%, and the boiling point was 135 - 144°C.

Water in weight ratios given in Table 2 was added to 10.0g of the methyl N,O-dimethylhydroxycarbamate obtained by the above method, and each solution was distilled using a five-stage Oldershow distillation column. Quantitatively analyzing the distillates by gas chromatography gave the results shown in Table 1.

**Table 1**

| Referential Example No. | Loading Composition N,O-dimethylhydroxycarbamate : Water (Ratio by Weight) | Pressure (mmHg) | Distillation Temperature (°C) | Distillate Composition N,O-dimethylhydroxycarbamate : Water (Ratio by Weight) |
|---|---|---|---|---|
| 1 | 1 : 0 | 760 | 144 | 100 : 0 |
| 2 | 1 : 1 | 760 | 96 | 49 : 51 |
| 3 | 1 : 5 | 760 | 97 | 47 : 53 |
| 4 | 1 : 5 | 80 | 41 | 45 : 55 |

## Claims

1. A process for recovering an N,O-dialkylhydroxycarbamic acid ester of formula (III) 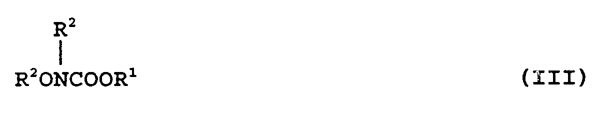 wherein R¹ is a hydrocarbon group, and R² is lower alkyl, said process comprising azeotropically distilling the N,O-dialkylhydroxycarbamic acid ester of formula (III) with water.

2. A process according to claim 1, wherein the N,O-dialkylhydroxycarbamic acid ester of formula (III) is azeotropically distilled with water from a solution containing the N,O-dialkylhydroxycarbamic acid ester of formula (III) and an O-alkylhydroxycarbamic acid ester of formula (IV)
R²ONHCOOR¹ (IV)
wherein R¹ and R² are as defined in claim 1.

3. A process according to claim 1 or 2, wherein the N,O-dialkylhydroxycarbamic acid ester of formula (III) is methyl N,O-dimethylhydroxycarbamate.

## Patentansprüche

1. Verfahren zur Gewinnung eines N,O-Dialkylhydroxycarbaminsäureesters der Formel (III) 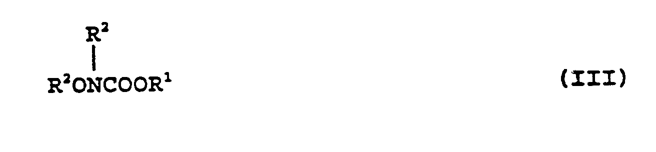 worin R¹ ein Kohlenwasserstoffrest und R² ein Niederalkylrest ist, umfassend das azeotrope Destillieren des N,O-Dialkylhydroxycarbaminsäureesters der Formel (III) mit Wasser.

2. Verfahren nach Anspruch 1, wobei der N,O-Dialkylhydroxycarbaminsäureester der Formel (III) mit Wasser aus einer Lösung azeotrop destilliert wird, die den N,O-Dialkylhydroxycarbaminsäureester der Formel (III) und einen O-Alkylhydroxycarbaminsäureester der Formel (IV)
R²ONHCOOR¹ (IV)
enthält, worin R¹ und R² wie in Anspruch 1 definiert sind.

3. Verfahren nach Anspruch 1 oder 2, wobei der N,O-Dialkylhydroxycarbaminsäureester der Formel (III) Methyl-N,O-dimethylhydroxycarbamat ist.

## Revendications

1. Procédé pour récupérer un ester d'acide N,O-dialkylhydroxycarbamique de formule (III) : 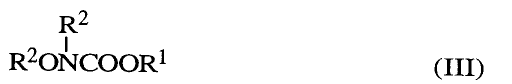 dans laquelle R¹ représente un groupe hydrocarboné et R² représente un groupe alkyle inférieur, ledit procédé comprenant la distillation azéotropique de l'ester d'acide N,O-dialkylhydroxycarbamique de formule (III) avec de l'eau.

2. Procédé conforme à la revendication 1, dans lequel on effectue la distillation azéotropique de l'ester d'acide N,O-dialkylhydroxycarbamique de formule (III) avec de l'eau, à partir d'une solution contenant l'ester d'acide N,O-dialkylhydroxycarbamique de formule (III) et un ester d'acide O-alkylhydroxycarbamique de formule (IV) :
R²ONHCOOR¹ (IV)
dans laquelle R¹ et R² sont tels que définis dans la revendication 1.

3. Procédé conforme à la revendication 1 ou 2, dans lequel l'ester d'acide N,O-dialkylhydroxycarbamique de formule (III) est le N,O-diméthylhydroxycarbamate de méthyle.
